Europäisches Patentamt

European Patent Office

Office européen des brevets

(11) Veröffentlichungsnummer: **0 343 406**
**A2**

## (12) EUROPÄISCHE PATENTANMELDUNG

(21) Anmeldenummer: **89108085.5**

(22) Anmeldetag: **05.05.89**

(51) Int. Cl.⁴: **A61K 37/54**

(30) Priorität: **27.05.88 DE 3818094**

(43) Veröffentlichungstag der Anmeldung:
**29.11.89 Patentblatt 89/48**

(84) Benannte Vertragsstaaten:
**AT BE CH FR GB IT LI LU NL SE**

(71) Anmelder: **MEDICHEMIE AG**
**Brühlstrasse 50**
**CH-4107 Ettingen/Basel(CH)**

(72) Erfinder: **Widauer, Josef Olaf, Dr. med.**
**Beim Lindenbaum 17**
**CH-4123 Allschwil(CH)**

(74) Vertreter: **Lauer, Joachim, Dr.**
**Hug Interlizenz AG Austrasse 44 Postfach**
**CH-8045 Zürich(CH)**

(54) **Verwendung von Lysozym zur Herstellung eines Nasenschleimhautpflegemittels.**

(57) Verwendung von Lysozym bzw. von Lysozymhydrochlorid zur Herstellung eines Nasenschleimhautpflegemittels zur Verbesserung der Funktion des Flimmerepithels, insbesondere zur Beförderung des Abtransports von Schleimhaut-Verunreinigungen in Richtung Rachen.

Verwendung von Lysozym in Nasentropfen, Sprays, Salben, Pulvern und/oder Pudern in Dosen von 0,1 bis 10 Milligramm pro Milliliter bzw. Gramm des Mittels.

EP 0 343 406 A2

## Verwendung von Lysozym zur Herstellung eines Nasenschleimhautpflegemittels

Die vorliegende Erfindung betrifft die Verwendung von Lysozym bzw. Lysozymhydrochlorid zur Herstellung eines Nasenschleimhautpflegemittels zur Verbesserung der Funktion des Flimmerepithels.

Durch verschiedene äussere Einwirkungen wie Luftverschmutzung, Rauch etc. oder innere Noxen, insbesondere Infektionen und Entzündungen der Atemwege oder durch degenerative Vorgänge kann es zu einer schädigenden Einwirkung auf die Nasenschleimhaut kommen. Damit vergesellschaftet ist häufig eine Funktionseinschränkung des sogenannten Flimmerepithels, welches normalerweise für den Abtransport von Schleimhaut-Verunreinigungen jeglicher Art in Richtung Rachen sorgt und somit eine wichtige Clearingfunktion ausübt. Bei Herabsetzen der Aktivität des Flimmerepithels kann es zu einer weiteren Anhäufung störender und zum Teil giftiger Stoffe kommen, die wiederum die Schleimhaut schädigen können. So kann ein Teufelskreis entstehen, der schliesslich zu einer Dauerschädigung (Atrophie) der Nasenschleimhaut mit all ihren unangenehmen Folgen führt.

Es wurde nun überraschend gefunden, dass durch künstliche Zufuhr von Lysozym in die Nasenhöhle die sekretomotorische Funktion des Flimmerepithels der Nasenschleimhaut verbessert wird, wobei ganz spezifisch die natürliche Sekretion in den Rachenraum beschleunigt und verstärkt wird.

Lysozym [Chemical Abstracts System Nr. 9001-63-2; E.C. (Enzyme Code) 3.2.1.17] ist bzw. Lysozyme sind Mucopeptidglucohydrolasen. Synonyme: Muramidase, N,O-Diacetylmuramidase, N-Acetyl-muramoyl-hydrolase. Lysozym kommt in vielen biologischen Geweben vor. Zu den reichsten Lysozymquellen gehören die menschlichen Leucozyten und das Hühnereiweiss. Lysozym ist in geringer Konzentration auch in Tränenflüssigkeit, Speichel, Serum, Milch, Milz, Nieren, Knochen, Placenta und Nasenschleim enthalten, wobei es offenbar eine Rolle bei der unspezifischen Immunabwehr spielt. Lysozym aus Hühnereiweiss wird aufgrund seiner bakteriolytischen Eigenschaften zur Behandlung von Infektionen verwendet. Es besteht aus einer Polypeptidkette aus 129 Aminosäuren mit einem Molekulargewicht von etwa 14'500, die durch 4 Disulfid- und zahlreiche Wasserstoffbrücken zu einem Sphäroprotein gefaltet ist.

Die Verstärkung der sekretomotorischen Funktion des Flimmerepithels der Nasenschleimhaut spezifisch gerichtet auf die Beschleunigung und Verstärkung der Sekretion in den Rachenraum war nicht bekannt. Ihre Entdeckung durch den Erfinder beruht auf einer Zufallsbeobachtung, die anschliessend im Doppelblindversuch wissenschaftlich erhärtet wurde.

Doppelblindversuch:

14 gesunden Probanden wurden entweder dosierte Sprühstösse einer 5 mg/ml Lysozym enthaltenden, gepufferten wässrigen Lösung oder dasselbe Volumen des Vehikels ohne Lysozym in die Nase instilliert. Nach 10 Minuten wurde 1 mg einer Saccharin-Tablette auf die vordere Nasenmuschel appliziert.

Nun wurde die Zeit bestimmt, bis das Saccharin durch die Nasenhöhle in den Rachenraum und so auf die Geschmackszäpfchen der Zunge gelangte und als intensive Süsse wahrgenommen wurde. Am nächsten Tag wurde der Versuch wiederholt, wobei die Probanden, die beim ersten Versuch mit Lysozym-Lösung behandelt wurden, nun mit dem Placebo, d.h. mit dem Vehikel ohne Lysozym behandelt wurden oder umgekehrt.

Die Versuchsanlage war randomisiert, doppelblind, so dass Versuchsleiter und Probanden nicht wussten, wann jeweils mit Lysozym und wann mit leerem Vehikel behandelt wurde.

Die Ergebnisse sind in der nachstehenden Tabelle aufgeführt.

Tabelle

| Proband Nr. | Zeit in Minuten bis zur Wahrnehmung des süssen Geschmackes nach Behandlung mit | | Zeitdifferenz Δ Minuten zwischen Wahrnehmung der Süsse nach Lysozym und nach Vehikellösung-Behandlung |
|---|---|---|---|
| | Lysozym-Lsg. | Vehikel-Lösung | |
| 1 | 19 Min. | 32 Min. | Δ 13 Min. |
| 2 | 9 Min. | 14 Min. | 5 Min. |
| 3 | 12 Min. | 14 Min. | 2 Min. |
| 4 | 10 Min. | 24 Min. | 14 Min. |
| 5 | 13 Min. | 17 Min. | 4 Min. |
| 6 | 14 Min. | 18 Min. | 4 Min. |
| 7 | 11 Min. | 17 Min. | 6 Min. |
| 8 | 10 Min. | 19 Min. | 9 Min. |
| 9 | 16 Min. | 23 Min. | 7 Min. |
| 10 | 14 Min. | 18 Min. | 4 Min. |
| 11 | 13 Min. · | 18 Min. | 5 Min. |
| 12 | 11 Min. | 19 Min. | 8 Min. |
| 13 | 12 Min. | 18 Min. | 6 Min. |
| 14 | 10 Min. | 15 Min. | 5 Min. |
| Mittel (1-14) | 12 Min.26 Sek. (174 Min.) | 19 Min. (266 Min.) | Δ 6 Min. 34 Sek. (92 Min.) |

EP 0 343 406 A2

Auswertung:

Alle Probanden nahmen den süssen Geschmack des Saccharins nach Behandlung der Nasenschleimhaut mit Lysozym schneller wahr als nach Behandlung mit leerer Vehikellösung. Die Zeitdifferenz ist sehr gross und beträgt mehr als die Hälfte der Zeit bis zur Wahrnehmung der Süsse nach der Behandlung mit Lysozym.

Daraus ist der Schluss zu ziehen, dass sich durch Behandlung der Nasenschleimhaut mit Lysozym-Lösung die Funktion des Flimmerepithels (Ciliarapparates) verbessern lässt und somit der Transport von Fremdkörpern aus der Nase in den Rachenraum spezifisch beschleunigt wird.

Basierend auf der Entdeckung dieser spezifischen Wirkung des Lysozyms auf die Nasenschleimhaut des Menschen, kann dem Patienten ein neues wirksames Mittel in die Hand gegeben werden, mit dessen Hilfe er den Symptomen einer gestörten Schleimhaut- und Ciliarfunktion, wie Krustenbildung oder trockene Nase, entgegenwirken kann. Damit wird die Funktion der Nase bzw. der Nasenschleimhaut als Filter, das periodisch oder laufend von festgehaltenen Staubpartikeln und/oder tröpfchenförmigen Verunreinigungen der Atemluft befreit werden muss, auf natürliche Art erheblich verbessert oder wieder hergestellt. Das subjektive Wohlbefinden und gleichzeitig der Schutz gegen Infektionen durch die Atemluft wird verbessert und die schädliche Wirkung verunreinigter Luft auf den Organismus verringert.

An Schnupfen erkrankten Patienten kann durch die Behandlung der Nasenschleimhaut mit Lysozym geholfen werden, derart, dass der Abtransport einer dabei vermehrten Sekretion aus der Nase in den Rachenraum gefördert und die pathologische Ausscheidung durch die Naris nach aussen vermindert oder verhindert wird. Dies ist darum wichtig, weil die Sekretion durch die Nasenlöcher nach aussen für den Patienten unangenehm und unhygienisch ist und für Dritte die Gefahr einer Infektionsübertragung heraufbeschwört.

Die Erfindung betrifft demnach die Verwendung von Lysozym bzw. von Lysozymhydrochlorid zur Herstellung eines Nasenschleimhautpflegemittels zur Verbesserung der Funktion des Flimmerepithes. Als Wirkstoff wird hauptsächlich aus Hühnereiweiss gewonnenes Lysozym verwendet.

Die erfindungsgemässe Verwendung von Lysozym erfolgt in Form von Nasentropfen bzw. Dosiersprays, Salben, Pulvern und Pudern. Als Salben kommen auch Cremes, Geles, Emulgeles in Tuben oder speziellen Dosierapplikatoren in Betracht. Puder und Pulver können durch Schnupfen appliziert werden (Schnupfmittel). Bezüglich der Dosierung des Wirkstoffes ist zu berücksichtigen, dass nicht nur die Menge in Milligramm (mg) ausschlaggebend für die Wirkung von Lysozym ist, sondern auch der Gehalt an sogenannten Sugar Units (SU). 1 SU entspricht der enzymatischen Wirkung von 1 mg Lysozym-Hydrochlorid, das bei 25 °C und bei photometrischer Wellenlänge von 450 Nanometer eine Verminderung des optischen Dichte einer definierten Micrococcus-Lysodeicticus-Suspension um 0,001/Min. bewirkt.

Ein gutes Lysozym hat einen biologischen Wirkungsgrad von mindestens 22,500 SU/mg.

Unter dieser Voraussetzung liegt die Dosierung bei 0,1 bis 10 mg Lysozym pro Milliliter (ml) bzw. Gramm (g) der Darreichungsform mit einem optimalen Bereich von 0,5 bis 5 mg pro ml bzw. g. Lysozym wird gewöhnlich als Lysozym-hydrochlorid verwendet.

Die Präparate können auser dem Wirkstoff Lysozym, Lösungsmittel, Träger und/oder Füllstoffe noch andere Substanzen enthalten, die für die Pflege der Nasenschleimhaut nützlich sind, wie beispielsweise Meersalz oder bei Schnupfen schleimhautabschwellende Mittel und/oder Antibiotika oder Desinfizientien.

Als Lösungsmittel kommen in Betracht Wasser, physiologische Kochsalz-Lösung oder ölige Lösungen, die Glycerin, flüssige Polyethylenglycole oder partiell verestertes Glycerin oder Polyethylenglycol enthalten. Als Träger für Pulver und Puder können verwendet werden Stärke, Silikate, Stearate und dergl.

Als Grundlage für Salben und Cremes kommen in Betracht Vaseline, Paraffine, pflanzliche oder tierische Fette und Öle, Wachse, synthetische Glyceride, Carboxyvinylpolymere (Carbopol) und Polyalkylsiloxane. Besonders geeignet sind hydrophile Salben aus Gemischen von flüssigen und festen Macrogolen, das sind beispielsweise Polyethylenglycole, Polyethylenglycolester wie Poly(oxyethylenoxyadipoyl), Poly-(oxyethylenoxysuccinyl), Macrogelstearat, Macrogel-glycerol-hydrostearat. Cremes sind im vorliegenden Fall Zubereitungen aus einer lipophilen und einer wässrigen Phase, enthaltend Emulgatoren von Wasser-in-Öl-Typ wie Wollfett, Sorbitanester und Monoglyceride oder Natrium-oder Triethanolaminseifen, sulfatierte Fettalkohole, Polysorbate oder Carboxyvinylpolymere. Geles bestehen aus Zubereitungen von Wasser, Glycerin oder Propylenglycol mit geeigneten Quellstoffen wie Traganth, Stärke, Cellulosederivaten, Carboxyvinylpolymeren oder Magnesium-Aluminium-Silikaten.

Den Nasenpflegemitteln können noch Geruchs- und Geschmacksstoffe, wie beispielsweise Campfer, Eucalyptus- oder Nelkenöl sowie Antibiotika, wie beispielsweise Tyrothricin, Bacitracin oder Amphomycin

4

und/oder Desinfizientia, wie Benzethonium-chlorid oder Dequalinium-chlorid zugesetzt werden.
Die Erfindung wird durch die folgenden Beispiele illustriert.

Beispiel 1

| Nasenpflegelösung | |
|---|---|
| Lysozym-Hydrochlorid | 2,5 g |
| Dequalinium-chlorid | 0,005 g |
| Polyvinylpyrrolidon (Plasdon K30$^{(R)}$) | 10,0 g |
| Meersalz | 10,0 g |
| Campfer | 0,5 g |
| Eucalyptusöl | 0,05 g |
| Propylenglycol | 20,0 g |
| Bidestilliertes Wasser | ad 1'000,0 g |

Das Dequalinium-chlorid, Lysozym-Hydrochlorid und Meersalz wird in destilliertem Wasser gelöst und mit dem Polyvinylpyrrolidon versetzt. Campfer und Eucalyptusöl werden in Propylenglycol gelöst. Die beiden Lösungen werden gemischt, mit destilliertem Wasser auf 1'000 g aufgefüllt und in Spray- oder Tropfflaschen abgefüllt.

Beispiel 2

| Nasensalbe | |
|---|---|
| Lysozym-Hydrochlorid | 0,25 kg |
| Cetylsalbe (Ung.cetyl) | 40,00 kg |
| Dünnflüssiges Paraffin | 10,00 kg |
| Weisses Vaselin | 40,00 kg |
| Dequalinium-chlorid | 0,5 g |
| Campfer | 0,05 kg |
| Eucalyptusöl | 5,0 g |
| Bidestilliertes Wasser | ad 100,00 kg |

Die Fettphase wird mit Eucalyptusöl und Campfer gemischt. Das Lysozym-Hydrochlorid und Dequalinium-chlorid wird in dest. Wasser gelöst und in der Fettphase emulgiert. Die Salbe wird in Tuben oder spezielle Applikatoren abgefüllt.

Beispiel 3

| Nasencreme | |
|---|---|
| Lysozym-Hydrochlorid | 2,5 g |
| Cetylan | 50,0 g |
| Gehärtetes Erdnussöl | 300,0 g |
| Propylenglycol | 100,0 g |
| Campfer | 0,5 g |
| Eucalyptusöl | 0,05 g |
| Dequalinium-chlorid | 0,005 g |
| Destilliertes Wasser | ad 1'000,0 g |

Die Fettphase wird geschmolzen und mit Campfer sowie Eucalyptusöl versetzt. Das Lysozym und Dequlinium-chlorid werden im Wasser gelöst und mit der Fettphase vereinigt und kalt gerührt. Die erhaltene Creme wird in Tuben, Töpfe oder spezielle Applikatoren abgefüllt.

Beispiel 4

| Nasengel | |
|---|---|
| Lysozym-Hydrochlorid | 25,00 g |
| Dequalinium-chlorid | 0,05 g |
| Natrium carboxymethylallulose | 300,00 g |
| Propylenglycol | 200,00 g |
| Campfer | 5,00 g |
| Eucalyptusöl | 0,50 g |
| Destilliertes Wasser | ad 10,00 kg |

Lysozym und Dequalinium-chlorid werden im Wasser gelöst, die Lösung wird mit der Natriumcarboxymethylcellulose versetzt und gerührt. Campfer und Eucalyptusöl werden im Propylenglycol gelöst und mit dem wässrigen Gel vermischt. Das erhaltene Nasengel wird in zur Applikation geeignete Behälter abgefüllt.

Beispiel 5

| Schnupfpulver | |
|---|---|
| Lysozym-Hydrochlorid | 2,5 g |
| Campfer | 0,5 g |
| Eucalyptusöl | 0,05 g |
| Reisstärke | ad 1'000,00 g |

Campfer und Eucalyptusöl werden mit etwa der Hälfte der Stärke angerieben. Die andere Hälfte der Stärke wird zum Anreiben des Lysozyms benutzt. Beide Teile werden vereinigt, homogenisiert, gemischt und in geeignete Behälter abgefüllt.

Beispiel 6

Schnupfenmittel

Einer analog Beispiel 2 erhaltenen Salbe werden anstelle von Dequalinium-chlorid dieselbe Menge Benzethoniumchlorid sowie 1 g Tyrothricin zugesetzt.

Die in den vorstehenden Beispielen beschriebenen Nasenschleimhautpflegemittel werden in Dosen von etwa 2 bis 10 Tropfen bzw. 0,1 bis etwa 1 g Salbe, Creme oder Gel durch die Naris in die Nase appliziert. Pulverförmige Darreichungsformen können durch die Naris eingeschnupft werden.

**Ansprüche**

1. Verwendung von Lysozym bzw. Lysozymhydrochlorid zur Herstellung eines Nasenschleimhautpflegemittels zur Verbesserung der Funktion des Flimmerepithels.

2. Verwendung nach Anspruch 1 von aus Hühnereiweiss gewonnenem Lysozym.

3. Verwendung nach Anspruch 1 von Lysozym in Nasentropfen, Sprays, Salben, Pulvern und/oder Pudern in Dosen von 0,1-10 Milligramm pro Milliliter bzw. Gramm des Mittels.